# EUROPÄISCHE PATENTSCHRIFT

(11) **EP 3 606 630 B1**
(45) Veröffentlichungstag und Bekanntmachung des Hinweises auf die Patenterteilung: **10.03.2021**
(21) Anmeldenummer: 18713264.2
(22) Anmeldetag: 29.03.2018
(51) Int. Cl.: B01D 3/00, C07C 265/14, C01B 32/80, C07C 263/20, C07C 263/10, B01J 12/00, B01D 1/00, B01D 1/16, B01D 1/20, B01D 53/14, B01D 53/00

(54) **REINIGUNGSVORRICHTUNG FÜR GASSTRÖME AUS DER ISOCYANATHERSTELLUNG**
CLEANING DEVICE FOR GAS FLOWS FROM ISOCYANATE PRODUCTION
DISPOSITIF DE NETTOYAGE POUR DES FLUX DE GAZ ISSUS DE LA FABRICATION D'ISOCYANATE

(30) Priorität: 03.04.2017 EP 17164492; 12.04.2017 LU 100164
(43) Veröffentlichungstag der Anmeldung: 12.02.2020
(73) Patentinhaber: Covestro Intellectual Property GmbH & Co. KG, 51373 Leverkusen (DE)
(72) Erfinder: MERKEL, Michael, 40223 Düsseldorf (DE); KOBYLKA, Manfred, 51399 Burscheid (DE); DÜRIG, Dominik, 52066 Aachen (DE); SEIDEL, Bernhard, 51381 Leverkusen (DE)
(74) Vertreter: Levpat
(86) Internationale Anmeldenummer: PCT/EP2018/058089
(87) Internationale Veröffentlichungsnummer: WO 2018/184980

(56) Entgegenhaltungen:
- WO-A1-2011/003532
- WO-A2-2007/014936

## Beschreibung

Die Erfindung betrifft eine Reinigungsvorrichtung zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt, umfassend eine erste Abtrenneinrichtung, die wenigstens eine Rohproduktzuleitung für einen mindestens das Isocyanat, Chlorwasserstoff und nicht umgesetztes Phosgen enthaltenden gasförmigen Rohproduktstrom, eine erste Flüssigkeitszuleitung für einen mindestens eine Quenchflüssigkeit enthaltenden Flüssigkeitsstrom und eine erste Flüssigkeitsableitung für einen mindestens einen Teil der Quenchflüssigkeit und einen Teil des Isocyanats enthaltenden Flüssigkeitsstrom aufweist, wobei von der ersten Abtrenneinrichtung eine Gasleitung zur Beförderung eines mindestens Chlorwasserstoff, verdampfte Quenchflüssigkeit und Phosgen enthaltenden Gasstroms abgeht. Außerdem betrifft die Erfindung ein Verfahren zur Reinigung von organische Isocyanate enthaltenden Produktströmen aus einer Quenchzone der Gasphasenphosgenierung. Weiterhin offenbart ist ein gasförmiges Gemisch zur Behandlung in einer Waschkolonne.

Die Herstellung von organischen Isocyanaten, insbesondere Diisocyanaten, in der Gasphase ist schon seit längerem im Stand der Technik allgemein beschrieben und wird industriell, insbesondere zur Herstellung von Toluylendiisocyanat, 1,6-Hexamethylendiisocyanat, Isophorondiisocyanat und Diisocyanatodicyclohexylmethan, genutzt. In allen Verfahren fällt ein gasförmiges Rohprodukt an, dass mindestens Isocyanat, Chlorwasserstoff und nicht umgesetztes Phosgen (Phosgen wird stets im Überschuss eingesetzt) umfasst, und das weiter aufgearbeitet werden muss, um das gewünschte Isocyanat in Reinform zu gewinnen.

Die WO 2007/014936 A2, Verfahren zur Herstellung von Isocyanaten (in der Gasphase), beschreibt eine Quenchzone, in welcher durch Eindüsen einer Quenchflüssigkeit das gasförmige Rohprodukt rasch abgekühlt wird. In dieser Quenchzone wird das Reaktionsgemisch, das im Wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570 °C auf 100 bis 200 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im Wesentlichen vollständig in der Gasphase verbleiben. Als mögliche Quenchflüssigkeiten werden Lösungsmittel, Isocyanat-Gemische und Lösungsmittel-Isocyanat-Gemische genannt. Erwähnt wird das Eindüsen einer Quenchflüssigkeit zum Abkühlen des Reaktionsgemisches und selektiven Lösen des gebildeten Isocyanats im Lösungsmittel, wobei eine erste Trennung in eine Flüssigphase und eine Gasphase mit überwiegend Phosgen und Chlorwasserstoff als Bestandteilen erfolgt. Die beiden Phasen werden danach einer entsprechenden Aufarbeitung zugeführt. Auf weiterhin wünschenswerte Optimierungsmöglichkeiten dieses Verfahrensschrittes wird nicht eingegangen.

Die WO 2014/122180 A1 offenbart ein Verfahren zur Abtrennung eines durch Umsetzung eines primären Amins mit einem Überschuss an Phosgen in der Gasphase hergestellten Isocyanats aus dem in der Umsetzung erhaltenen gasförmigen Rohprodukt, bei dem der Volumenstrom zur Waschkolonne durch Einsatz eines vorgeschalteten Wärmetauschers bzw. Kondensators verringert und somit eine Waschkolonne geringen Durchmessers und mit einer geringen Trennstufenzahl ausreichend ist. Zwar lässt sich die Waschkolonne verkleinern, jedoch kann es zu Ablagerungen im Kondensator kommen, die eine Reinigung und somit eine Unterbrechung des kontinuierlichen Prozesses erfordern. Zudem ist die Installation des Kondensators, dessen Betrieb und Wartung mit zusätzlichem Aufwand verbunden. Je nach Zusammensetzung des gasförmigen Rohprodukts muss mit dessen korrosiven Eigenschaften gerechnet werden, was den Aufwand beziehungsweise die Materialanforderungen für den Kondensator weiter erhöht.

Bei niedrigen Kondensationstemperaturen kommt es zudem verstärkt zur unerwünschten Kondensation von Phosgen aus dem Gasstrom und damit zu einer Erhöhung der in der Anlage vorhandenen Phosgenmenge. Dies ist im Sinne einer effizienten Aufbereitung des Phosgens unerwünscht. So wird z.B. in WO 2011/003532 A1 auf die Aufbereitung des überwiegend aus Phosgen und Chlorwasserstoff enthaltenden Gasstroms eingegangen.

Daher war es die Aufgabe der vorliegenden Erfindung eine Reinigungsvorrichtung zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt bereitzustellen, mit der die Bildung von Ablagerungen in der Waschkolonne sowie in zwischen der ersten Abtrenneinrichtung (Quenche) und der Waschkolonne befindlichen Apparaten und Rohrleitungen deutlich verzögert werden kann und die Kondensation von Phosgen aus dem Gasstrom weitestgehend vermieden wird.

Diese Aufgabe wird erfindungsgemäß dadurch gelöst, dass der ersten Gasleitung (9) mindestens eine Zugabeeinrichtung (11) zum unmittelbaren Einbringen mindestens eines Kühlfluids für eine wenigstens teilweise Kondensation und/oder Absorption des durch die erste Gasleitung (9) führbaren Gasstroms zugeordnet ist. Eine Verringerung des Volumenstroms durch Kondensation der gasförmigen Bestandteile ist hierbei nicht zwangsläufig angestrebt. Es ist vielmehr erwünscht, dass Phosgen weitestgehend in der Gasphase verbleibt, um die in der Anlage vorhandene Phosgenmenge möglichst klein zu halten. Je nach Betriebsweise kann es durch die Zugabe des Kühlfluids sogar zu einer (leichten) Erhöhung des Volumenstroms kommen. Entscheidend für die Erfindung ist vielmehr eine Vorreinigung des gasförmigen Produktstroms ohne zwangsläufige Beeinflussung des Volumenstroms.

Somit ist ein erster Gegenstand der Erfindung eine Reinigungsvorrichtung (1) zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt, umfassend eine erste Abtrenneinrichtung (2), die wenigstens
- eine Rohproduktzuleitung (3) für einen mindestens das Isocyanat, Chlorwasserstoff und nicht umgesetztes Phosgen enthaltenden gasförmigen Rohproduktstrom,
- eine erste Flüssigkeitszuleitung (4) für einen mindestens eine Quenchflüssigkeit enthaltenden Flüssigkeitsstrom und
- eine erste Flüssigkeitsableitung (5) für einen mindestens einen Teil der Quenchflüssigkeit und einen Teil des Isocyanats enthaltenden Flüssigkeitsstrom aufweist,
wobei von der ersten Abtrenneinrichtung (2) eine erste Gasleitung (9) zur Beförderung eines mindestens Chlorwasserstoff, verdampfte Quenchflüssigkeit und Phosgen enthaltenden Gasstroms abgeht,
dadurch gekennzeichnet, dass der ersten Gasleitung (9) mindestens eine Zugabeeinrichtung (11) zum unmittelbaren Einbringen mindestens eines Kühlfluids für eine wenigstens teilweise Kondensation und/oder Absorption des durch die erste Gasleitung (9) führbaren Gasphasenstroms zugeordnet ist.

Vorliegend wird unter Kühlfluid bevorzugt ein flüssiges Medium verstanden, welches vorzugsweise frei von Verbindungen ist, die Zerewitinoff-aktive Gruppen aufweisen, welche mit Isocyanat reagieren würden. Bevorzugte Kühlfluide sind beispielsweise die im Folgenden genannten Quenchflüssigkeiten. Je nach bevorzugter Ausführungsform kann das Kühlfluid auch zumindest teilweise aus dem Prozess entnommen werden, beispielsweise als Flüssigkeit aus einer zweiten Abtrenneinrichtung (10). Besonders bevorzugt handelt es sich um eine Quenchflüssigkeit, die auch zum Abkühlen des aus der Reaktionszone austretenden Reaktionsgemisches verwendet wird, wobei der Gehalt an Isocyanat im Kühlfluid bevorzugt ≤ 20 Gew.-%, besonders bevorzugt ≤ 10 Gew.-% und ganz besonders bevorzugt ≤ 2 Gew.-% ist.

Vorliegend wird unter dem, durch die erste Gasleitung (9) förderbaren, mindestens Chlorwasserstoff, verdampfte Quenchflüssigkeit und Phosgen enthaltenden Gasstrom, ein Gasstrom verstanden, der auch ein Aerosol darstellen kann, welches einen geringen Anteil an Flüssigkeitströpfchen und/oder Feststoffpartikeln enthält. Außerdem kann der Gasstrom auch noch kleinere Mengen des Isocyanats enthalten, welche nicht vollständig in der ersten Abtrenneinrichtung (2) abgetrennt wurden. Auch wenn es besonders stromabwärts nach der erfindungswesentlichen Zugabeeinrichtung (11) zu einer vermehrten Flüssigkeitsbildung kommen kann und somit eine Gasphase und eine flüssige Phase vorliegen, wird dieses durch die erste Gasleitung (9) geführte oder führbare, zweiphasige Gemisch im Nachfolgenden zusammenfassend als Gasstrom bezeichnet, da die flüssigen Anteile teilweise bereits in gasförmiger Form von der ersten Abtrenneinrichtung (2) in die erste Gasleitung (9) gelangt sind.

Vorliegend wird unter "Quenchzone" ein Bereich verstanden, in welchem durch Eindüsen einer Quenchflüssigkeit das gasförmige Rohprodukt rasch abgekühlt wird. In dieser Quenchzone wird das Reaktionsgemisch, das im Wesentlichen aus dem Isocyanat, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 600 °C auf 100 bis 200 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im Wesentlichen vollständig in der Gasphase verbleiben.

Die erste Abtrenneinrichtung (2) kann vorliegend als Quenchzone verstanden werden. So ist die erste Abtrenneinrichtung (2) dazu eingerichtet, den durch die Rohproduktzuleitung (3) führbaren Gasstrom durch Inkontaktbringen mit, bevorzugt durch Eindüsen, einer Quenchflüssigkeit rasch abzukühlen und teilweise zu verflüssigen ("Quenche"). Geeignete erste Abtrenneinrichtungen (2) sind beispielsweise in EP 1 403 248 A1, siehe insbesondere die Zeichnungen mit den zugehörigen Erläuterungen in den Absätzen [0017] bis [0019], und EP 1 935 875 A1, siehe insbesondere die Absätze [0015] bis [0022] und [0033] bis [0045], beschrieben. Für die Beförderung der Quenchflüssigkeit weist die Abtrenneinrichtung (2) eine erste Flüssigkeitszuleitung (4) auf.

Als durch die erste Flüssigkeitszuleitung (4) führbare Quenchflüssigkeiten sind Lösungsmittel wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol, Xylole, oder deren Mischungen geeignet. Bevorzugte Lösungsmittel sind Chlorbenzol, o-Dichlorbenzol und/oder p-Dichlorbenzol und besonders bevorzugt sind Chlorbenzol oder o-Dichlorbenzol. Dieser zusätzliche Strom an frischem, recycliertem und/oder aufbereitetem organischem Lösungsmittel hat bevorzugt eine Temperatur von 40 °C bis 150 °C.

Die erste Abtrenneinrichtung weist eine erste Flüssigkeitsableitung (5) für einen mindestens einen Teil der Quenchflüssigkeit und einen Teil des Isocyanats enthaltenden Flüssigkeitsstrom auf. Hierbei ist ein möglichst hoher Anteil erstrebenswert, bevorzugt ≥ 80 %, besonders bevorzugt ≥ 90 %, besonders bevorzugt ≥ 98% und ganz besonders bevorzugt ≥ 99% bis ≤ 99,95% des im Rohproduktstrom enthaltenen Isocyanats. Bei den vorstehend genannten Anteilen ist zu beachten, dass durch den Dampfdruck des Isocyanats eine natürliche Grenze für die maximale Abtrennung des Isocyanats gesetzt ist und der wirtschaftliche Aufwand gegen den Nutzen einer möglichst starken Annäherung an diese Grenze abgewogen werden muss. Der in diesem Schritt erhaltene Gasstrom kann deshalb in der Regel noch geringe Mengen an Isocyanat enthalten.

Bevorzugt ist, dass von der ersten Flüssigkeitsableitung (5) eine erste Flüssigkeitsleitung (6) für die Rückförderung eines Teils des durch die erste Flüssigkeitsableitung (5) führbaren Flüssigkeitsstroms abgeht und in die erste Flüssigkeitszuleitung (4) mündet. Falls erforderlich, kann der ersten Flüssigkeitsleitung (6) eine Pumpe (7) zur Zwangsförderung dieses Flüssigkeitsstroms zugeordnet sein. Auch möglich ist es, das der ersten Flüssigkeitsleitung (6) ein Quenchekühler (8) zur Kühlung des durch die zweite Flüssigkeitsleitung führbaren Flüssigkeitsstroms zugeordnet sein kann.

Bevorzugt bedeuten erfindungsgemäß die Ausdrücke "umfassend" oder "enthaltend", "im Wesentlichen bestehend aus" und besonders bevorzugt "bestehend aus".

Die Phosgenierung von organischen Aminen in der Gasphase an sich ist bekannt und kann beispielsweise erfolgen, wie beschrieben in EP 0 289 840 B1, EP 1 319 655 A2, EP 1 555 258 A1, EP 1 275 639 A1, EP 1 275 640 A1, EP 1 449 826 A1, EP 1 754 698 B1, DE 10 359 627 A1 oder DE 10 2005 042392 A1. Geeignete organische Amine sind beispielsweise Anilin, Pentamethylendiamin (nachfolgend auch PDA), Hexamethylendiamin (nachfolgend auch HDA), die Isomere des Isophorondiamins (nachfolgend auch IPDA), die Isomere des Xylylendiamins (nachfolgend auch XDA), die Isomere des Diaminodicyclohexylmethans (nachfolgend auch H12-MDA), die 2-kernigen Isomere des Methylendiphenyldiamins (nachfolgend auch MDA) sowie die Isomere des Toluylendiamins (nachfolgend auch TDA). Bevorzugt sind TDA, PDA, HDA, IPDA und XDA, besonders bevorzugt sind TDA, PDA, HDA und IPDA, ganz besonders bevorzugt sind TDA und HDA.

Das organische Amin wird mit Phosgen in kontinuierlicher Betriebsweise umgesetzt. Hierbei erfolgt die Umsetzung bevorzugt bei einer Temperatur von 200 °C bis 600 °C, bevorzugt von 300 °C bis 500 °C, und einem absoluten Druck von 150 mbar bis 10 bar, bevorzugt von 1,0 bar bis 3,0 bar. Der molare Überschuss an Phosgen beträgt bevorzugt 20% bis 400% der Theorie. Der aus der Umsetzung von organischem Amin mit Phosgen erhaltene Gasstrom enthält mindestens das Isocyanat, Chlorwasserstoff und nicht umgesetztes Phosgen und ist als Rohproduktstrom durch die Rohproduktzuleitung (3) führbar.

Gemäß einer ersten bevorzugten Ausführungsform der Erfindung sind die erste Abtrenneinrichtung (2) und/oder die erste Gasleitung (9) so eingerichtet, dass der durch die erste Gasleitung (9) führbare Gasstrom vor dem Erreichen der Zugabeeinrichtung (11) mindestens einmal, bevorzugt mindestens zweimal und besonders bevorzugt zwei bis fünfmal umgelenkt wird. Dies hat den Vorteil, dass ein größerer Anteil eventuell mitgerissener Flüssigkeitströpfchen abgeschieden werden kann. Die Umlenkungen können dabei unabhängig voneinander in einem Behälter erfolgen, wobei Ein- und Austrittsrohr nicht an gegenüberliegenden Seiten des Behälters angeordnet sind und/oder mittels in der ersten Gasleitung (9) eingebauten Rohrbögen. Die erste Umlenkung kann dabei bereits beim Übergang aus der ersten Abtrenneinrichtung (2) in die Gasleitung (9) erfolgen.

Gemäß einer weiteren bevorzugten Ausführungsform der Erfindung besteht die Zugabeeinrichtung (11) aus einer oder mehreren Düsen, die zum unmittelbaren Einbringen des Kühlfluids im Gleich- oder im Gegenstrom, bevorzugt im Gleichstrom, eingerichtet sind oder, ebenfalls bevorzugt, aus mehreren Düsen, die im Leitungsmantel der ersten Gasleitung (9), besonders bevorzugt in gleichmäßigen Abständen entlang des Umfangs der ersten Gasleitung (9), positioniert sind. Hieraus ergibt sich der Vorteil, dass ein feiner Nebel erzeugt wird, der zu einer weiter verbesserten Agglomeration der Aerosolpartikel führt.

Besonders bevorzugt sind die eine oder mehrere Düsen so angeordnet, dass der erzeugte Nebel den gesamten Rohrquerschnitt abdeckt. Geeignete Düsen sind aus dem Stand der Technik bekannt. Es können z.B. einfache Lochdüsen, aber auch solche mit Einbauten oder Zweistoff-Düsen verwendet werden. Bevorzugt sind Einstoff-Düsen, um eine zusätzliche Gasbelastung der nachfolgenden Apparate zu vermeiden.

In einer ganz besonders bevorzugten Ausführungsform der Erfindung befindet sich die Zugabeeinrichtung (11) in einem vertikal verlaufenden Rohrleitungsteil, in dem das gasförmige Rohprodukt von oben nach unten strömt.

In einer weiteren Ausführungsform können auch mehrere solcher Zugabeeinrichtungen (11) in Strömungsrichtung des Gases hintereinander angeordnet sein.

Alternativ zur vorgenannten bevorzugten Ausführungsform ist es auch bevorzugt, wenn die Zugabeeinrichtung (11) mindestens eine, bevorzugt mehrere Öffnungen zum Erzeugen eines Fluidvorhangs über den gesamten Querschnitt der ersten Gasleitung (9) ist, die in einem horizontal verlaufenden Rohrleitungsteil im oberen Bereich des Leitungsmantels der ersten Gasleitung (9) positioniert ist, so dass der Gasstrom die Lamellen oder Strahlen des Fluidvorhangs kreuzt. Dabei ist es möglich, dass die Öffnungen rund oder schlitzförmig ausgestaltet sind, oder es sich z.B. um Flachstrahldüsen handelt. Möglich ist auch die Führung des Fluidvorhangs durch eine in der Rohrleitung angebrachte Koaleszenzhilfe. Als horizontal verlaufende Rohrleitungsteile sind im Sinne der Erfindung solche Rohrleitungen zu betrachten, die weniger als 60% Gefälle, bevorzugt weniger als 10% Gefälle, besonders bevorzugt 0,01 - 2% Gefälle aufweisen. Hieraus ergibt sich der Vorteil, dass auch auf diese Weise die Wahrscheinlichkeit zur Agglomeration der Aerosolpartikel weiter erhöht wird.

Von den beiden vorstehend genannten, alternativen bevorzugten Ausführungsformen ist die erste Ausführungsform besonders bevorzugt, da diese unter manchen Umständen besonders effektiv sein kann.

Gemäß einer weiteren bevorzugten Ausführungsform weist die Zugabeeinrichtung (11) eine Kühlfluidzuleitung (12) zur Beförderung des Kühlfluids auf.

In Weiterbildung der Erfindung ist es vorgesehen, dass die erste Gasleitung (9) zu einer zweiten Abtrenneinrichtung (10) führt.

Hierbei ist es weiter bevorzugt, dass von der zweiten Abtrenneinrichtung (10) eine zweite Flüssigkeitsleitung (13) zur zumindest teilweisen Rückführung eines Flüssigkeitsstroms als Quenchflüssigkeit abgeht und zur ersten Abtrenneinrichtung (2) hinführt. Falls erforderlich kann dieser Flüssigkeitsstrom, beispielsweise mittels einer Pumpe (14), zwangsgefördert werden.

Die Kühlfluidzuleitung (12) kann unter anderem direkt einem Vorlagebehälter für das Kühlfluid entspringen. Bevorzugt entspringt sie als Abzweig aus der zweiten Flüssigkeitsleitung (13), so dass beide Leitungen ((12) und (13)) über eine gemeinsame Pumpe (14) versorgt werden können.

Hierbei ist es besonders bevorzugt, dass der zweiten Flüssigkeitsleitung (13) und der Kühlfluidzuleitung (12) zusätzlich Regelungseinrichtungen (15a) und (15b) zur Steuerung der durch die zweite Flüssigkeitsleitung (13) zur ersten Abtrenneinrichtung (2) einerseits und durch die Kühlfluidzuleitung (12) zur Zugabeeinrichtung (11) andererseits führbaren Flüssigkeitsmengenströme zugeordnet sind. Solche Regelungseinrichtungen sind aus dem Stand der Technik hinlänglich bekannt. Es können einfache Handarmaturen sein, über die eine Begrenzung des freien Leitungsquerschnitts eingestellt und somit der Volumenstrom begrenzt werden kann. Bevorzugt, wird eine Kombination aus Sensor und Aktor eingesetzt, die über eine Regelungsautomatik die jeweiligen Mengenströme selbstständig auf einen vorgegebenen Wert regelt. Als Sensoren können beispielsweise Volumendurchflussmesser wie Magnetisch-induktive Durchflussmesser, Schwebekörper-Durchflussmesser, Ultraschall-Durchflussmesser, Messblenden, Staudrucksonden oder Massendurchflussmesser wie Coriolis-Massendurchflussmesser, thermische Massendurchflussmesser oder Vortex-Massendurchflussmesser verwendet werden. Als Aktoren dienen z.B. über Motoren angetriebene Schieber, Drehkegelventile, Kugelhähne, Kolbenventile oder Absperrklappen. Hieraus ergibt sich der Vorteil, dass sich die Temperaturen in der ersten Abtrenneinrichtung (2) und in der Zugabeeinrichtung (11) durch die Menge an Kühlfluid steuern lassen und dass die Menge an zusätzlichem Kühlfluid optimal niedrig eingestellt werden kann, so dass eine hinreichende Vorreinigung erzielt wird, aber der Aufwand zur Aufbereitung oder Beschaffung des zusätzlichen Kühlfluids minimiert wird.

Um einen zusätzlichen Freiheitsgrad bei der Einstellung der geeigneten Betriebsparameter zu erhalten, kann der Kühlfluidzuleitung (12) und/oder der zweiten Flüssigkeitsleitung (13) ein Wärmetauscher (16) für die Temperierung der durch die Leitungen führbaren Flüssigkeitsströme zugeordnet sein. Geeignete Wärmetauscher und deren Betriebsweise sind dem Fachmann aus dem Stand der Technik bekannt, es können unter anderem Rohrbündelwärmetauscher oder Plattenwärmetauscher eingesetzt werden.

Die zweite Abtrenneinrichtung (10) kann eine vierte Flüssigkeitsleitung (21) zur Zugabe eines Flüssigkeitsstroms aufweisen. Bevorzugt weist die zweite Abtrenneinrichtung (10) eine zweite Gasleitung (17) zur Ableitung des nicht-kondensierten Gasstroms auf, die ganz besonders bevorzugt im oberen Teil der zweiten Abtrenneinrichtung (10) abgeht. Der zweiten Gasleitung (17) ist ein Kondensator (18) zur zumindest teilweisen Kondensation des durch zweite Gasleitung (17) führbaren Gasstroms zugeordnet. Weiterhin weist der Kondensator (18) eine dritte Flüssigkeitsleitung (20) zur Rückführung des kondensierten Flüssigkeitsstroms auf, die in die zweite Abtrenneinrichtung (10) mündet.

In einer weiteren Ausführungsform kann eventuell anfallende Flüssigkeit des durch die erste Gasleitung (9) führbaren Gasstroms in den unteren Teil der ersten Abtrenneinrichtung (2) abgeleitet werden, bevorzugt ist jedoch, dass die erste Gasleitung (9) von der Zugabeeinrichtung (11) zur zweiten Abtrenneinrichtung (10) mit Gefälle verläuft, so dass die Gravitation genügend Triebkraft bietet, um anfallende Flüssigkeit zur zweiten Abtrenneinrichtung (10) zu befördern und auf diese Weise Flüssigkeitsansammlungen im Bereich der Zugabeeinrichtung (11) zu vermeiden.

Die zweite Abtrenneinrichtung (10) ist vorzugsweise eine Waschkolonne mit mindestens einer Trennstufe, wobei der Waschkolonne mindestens ein Lösungsmittelstrom als Waschflüssigkeit zugeführt werden kann. Geeignete Waschkolonnen (10) sind beispielsweise beschrieben in Perry's Chemical Engineers' Handbook, 7th Edition, McGraw-Hill, Kapitel 14 "Gas Absorption and Gas-Liquid System Design". Bevorzugt enthält der durch die dritte Flüssigkeitsleitung (20) und/oder durch die vierte Flüssigkeitsleitung (21) führbare Flüssigkeitsstrom chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und Xylole. Besonders bevorzugte Lösungsmittel sind Chlorbenzol und die Isomeren des Dichlorbenzols, ganz besonders bevorzugt sind Chlorbenzol oder o-Dichlorbenzol. Der mindestens eine Lösungsmittelstrom kann aus frischem Lösungsmittel bestehen. Es ist jedoch auch möglich rezyklierte, gegebenenfalls aufbereitete Lösungsmittelhaltige Ströme oder Mischungen der vorgenannten Lösungsmittel einzusetzen.

In einer weiteren bevorzugten Ausführungsform ist die vierte Flüssigkeitsleitung (21) oberhalb der Trennstufe der Waschkolonne (10), bei Vorhandensein mehrerer Trennstufen oberhalb der obersten Trennstufe, angeordnet. Wenn als Lösungsmittelstrom nicht frisches Lösungsmittel, sondern ein rezyklierter Lösungsmittel-haltiger Strom eingesetzt werden soll, ist darauf zu achten, dass dieser im Wesentlichen frei von dem abzutrennenden organischen Isocyanat ist, vorzugsweise das abzutrennende organische Isocyanat in einem Massenanteil von maximal 100 ppm, bezogen auf die Gesamtmasse des durch die vierte Flüssigkeitsleitung (21) führbaren Flüssigkeitsstroms, enthält, um die Bildung von Zersetzungsprodukten zu vermeiden.

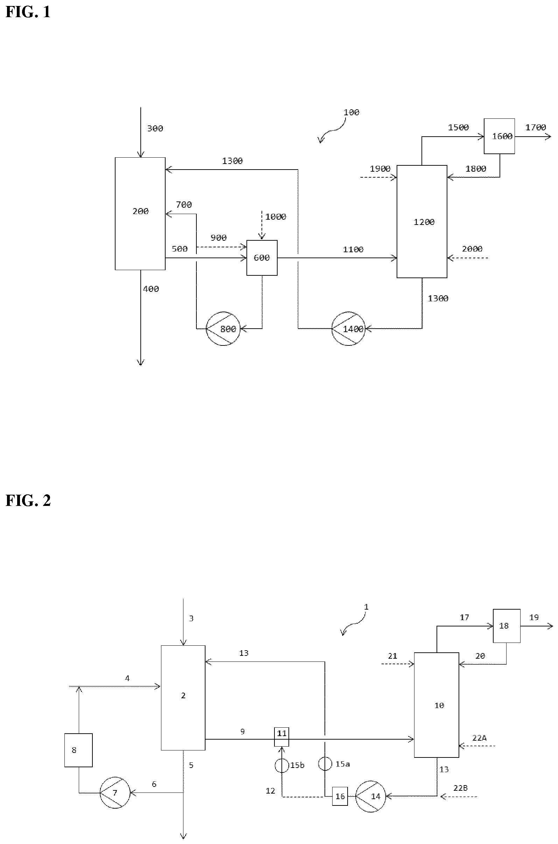

Alternativ zu oder in Kombination mit dieser Ausführungsform kann die zweite Abtrenneinrichtung (10), bevorzugt die Waschkolonne (10), eine fünfte Flüssigkeitsleitung (22) zur Zugabe eines Flüssigkeitsstroms aufweisen, die unterhalb der Trennstufe, bei Vorhandensein mehrerer Trennstufen unterhalb der untersten Trennstufe, zugeführt werden. Die Einbindung kann in die Abtrenneinrichtung selbst (beispielhaft in FIG. 2 als fünfte Flüssigkeitsleitung (22A) schematisch dargestellt) und/oder in das zugehörige Ablaufrohr für Flüssigkeiten (13) erfolgen (beispielhaft in FIG. 2 als fünfte Flüssigkeitsleitung (22B) schematisch dargestellt), bei Verwendung eines Zwangsförderers bevorzugt auf dessen Saugseite, also in die zweite Abtrenneinrichtung und/oder zwischen zweiter Abtrenneinrichtung und Zwangsförderer. Diese Ausführungsform bietet den Vorteil einer verbesserten Durchmischung der Flüssigkeitsströme. Wenn der durch die fünfte Flüssigkeitsleitung (22) führbare Flüssigkeitsstrom nicht frisches Lösungsmittel, sondern ein rezyklierter Lösungsmittelhaltiger Strom ist, so sind hier die Anforderungen an den maximalen Isocyanat-Gehalt weniger streng als im Fall des durch die vierte Flüssigkeitsleitung (21) führbaren Flüssigkeitsstroms. Aufgrund des direkten Beitrags des letztgenannten Stroms zum Dampf-Flüssigkeits-Gleichwicht mit dem dampfförmigen Kopfproduktstrom der Waschkolonne, der weitgehend Isocyanat-frei sein muss, können in diesem Flüssigkeitsstrom nur sehr viel geringere Isocyanat-Anteile zugelassen werden als im durch die fünfte Flüssigkeitsleitung (22) führbaren Strom. Daher kann der durch die fünfte Flüssigkeitsleitung (22) führbare Flüssigkeitsstrom bis zu 20 Gew.-% Isocyanat, bevorzugt bis zu 10 Gew.-% Isocyanat und besonders bevorzugt bis zu 2 Gew.-% Isocyanat bezogen auf die Gesamtmasse des durch die fünfte Flüssigkeitsleitung (22) führbaren Flüssigkeitsstroms, enthalten.

Der Kondensator (18) weist eine dritte Gasleitung (19) zur Beförderung des nicht-kondensierten Gasstroms auf. Dieser nicht-kondensierte Gasstrom besteht im Wesentlichen aus Chlorwasserstoffgas, Phosgen, weiteren Gasen, wie zum Beispiel Stickstoff und Kohlenmonoxid, und geringfügigen Mengen an Lösungsmittel und wird bevorzugt einer weiteren Aufarbeitung zugeführt, wo in der Regel Lösungsmittel, überschüssiges Phosgen und entstandenes Chlorwasserstoffgas voneinander getrennt werden. Lösungsmittel und überschüssiges Phosgen werden (getrennt voneinander) aus wirtschaftlichen Gründen bevorzugt wieder der Reaktion zugeführt. Der Chlorwasserstoff kann verschiedenen Einsatzmöglichkeiten zugeführt werden, wie zum Beispiel einer Oxychlorierung von Ethylen zu Ethylendichlorid oder einem Recyclingverfahren, welches Chlor wieder in den Isocyanatprozess zurückführt. Zu diesen Recyclingverfahren zählen die katalytische Oxidation von Chlorwasserstoff, z. B. nach dem Deacon-Verfahren, die Elektrolyse von gasförmigem Chlorwasserstoff sowie die Elektrolyse einer wässrigen Lösung von Chlorwasserstoff (Salzsäure).

Durch den Betrieb der Zugabeeinrichtung (11) im erfindungsgemäßen Verfahren kann die Standzeit bis eine Reinigung und damit eine Unterbrechung des kontinuierlichen Betriebs erforderlich wird, gegenüber dem Stand der Technik erheblich verlängert werden. Dies wird erreicht, da bei ansonsten gleichen Druck- und Temperaturbedingungen in der Zuleitung zur zweiten Abtrenneinrichtung (10) bereits eine stärkere Anreicherung von mitgeschleppten organischen Verunreinigungen in der flüssigen Phase des durch die erste Gasleitung (9) führbaren Gasstroms erfolgt.

Die Vorteile der vorliegenden Erfindung lassen sich durch einfache Verringerung der Kondensationstemperaturen im bekannten Kondensator des Standes der Technik nicht erreichen, da es an den sehr kalten Oberflächen des Kondensators schnell zu Ablagerungen kommt, wodurch entweder die Temperatur nicht mehr eingehalten werden kann oder gar der Kondensator verstopft und gereinigt werden muss und somit der Wartungsaufwand erhöht würde. Darüber hinaus kommt es beim Betrieb eines Kondensators wie z.B. in WO 2014/122180 A1 beschrieben bei tieferen Temperaturen zu einer unerwünschten Kondensation von Phosgen aus dem Gasstrom und damit zu einer deutlichen Erhöhung der in der Anlage vorhandenen Phosgenmenge. Dabei ist es unerheblich, ob noch eine Berieselung des Kondensators erfolgt oder nicht. Wünschenswert ist an dieser Stelle ein überwiegender Verbleib des Phosgens in der Gasphase, sodass es anschließend, bevorzugt wie in WO 2011/003532 A1 beschrieben weiter aufgereinigt und in den Prozess recycliert werden kann. Bevorzugt befinden sich nach der Zugabeeinrichtung (11) ≥ 90% des Phosgens in der Gasphase des Gasstroms, besonders bevorzugt ≥ 95% und ganz besonders bevorzugt ≥ 97% bis ≤ 99,5%.

Durch die erfindungswesentliche Zugabeeinrichtung (11) lässt sich bei Betrieb der Reinigungsvorrichtung (1) eine verbesserte Abtrennung eventuell in dem durch die erste Gasleitung (9) förderbaren Gasstrom vorhandener Flüssigkeits- und/oder Feststoffpartikel erreichen. Daher ist ein weiterer Gegenstand der vorliegenden Erfindung ein Verfahren zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt, umfassend die Schritte,
(i) mindestens teilweise Kondensation des Rohproduktstroms, der mindestens das Isocyanat, Chlorwasserstoff und nicht umgesetztes Phosgen enthält, durch Inkontaktbringen mit mindestens einem Flüssigkeitsstrom, der mindestens eine Quenchflüssigkeit enthält, in einer ersten Abtrenneinrichtung (2) unter Erhalt
   eines mindestens einen Teil der Quenchflüssigkeit und einen Teil des Isocyanats enthaltenden Flüssigkeitsstroms und
   eines mindestens Chlorwasserstoff, verdampfte Quenchflüssigkeit und Phosgen enthaltenden Gasstroms,
(ii) Ableitung des in Schritt (i) erhaltenen Flüssigkeitsstroms über eine erste Flüssigkeitsableitung (5) und des in Schritt (i) erhaltenen Gasstroms über eine erste Gasleitung (9) und
(iii) wenigstens teilweise Kondensation und/oder Absorption des in Schritt (ii) durch die erste Gasleitung (9) abgeleiteten Gasstroms,
dadurch gekennzeichnet, dass die wenigstens teilweise Kondensation und/oder Absorption in Schritt (iii) durch unmittelbares Einbringen mindestens eines Kühlfluids erfolgt, wobei das Kühlfluid über mindestens eine Zugabeeinrichtung (11), die der ersten Gasleitung (9) zugeordnet ist, unmittelbar in die erste Gasleitung (9) eingebracht wird.

In einer bevorzugten Ausführungsform des erfindungsgemäßen Verfahrens beträgt das Gewichts-Verhältnis des eingebrachten Kühlfluids zum durch die erste Gasleitung (9) geführten Gasstrom bevorzugt 1:100 bis 10:1, besonders bevorzugt 1:12 bis 1:1, ganz besonders bevorzugt 1:10 bis 1:2. Stromabwärts der Zugabe des Kühlfluids stellt sich durch Regelung des Gewichtsverhältnisses und gegebenenfalls der Kühlfluid-Temperatur eine Temperatur ein, die unterhalb der des in Schritt (i) erhaltenen Gasstroms liegt. Bei einem Druck von 1,5 bar(a) liegt die Temperatur bevorzugt zwischen 100 °C und 170 °C, besonders bevorzugt zwischen 110 und 150 °C und ganz besonders bevorzugt zwischen 110 und 128 °C. Auf diese Weise wird verhindert, dass zu viel Phosgen in die flüssige Phase übergeht und sich dadurch die in der Anlage befindliche Menge an Phosgen erhöht. Bei höherem oder niedrigerem Druck sowie in Abhängigkeit des verwendeten Kühlfluids bzw. der verwendeten Quenchflüssigkeit, können die bevorzugten Temperaturen entsprechend den Regeln der Thermodynamik höher oder tiefer liegen. Als weitere bevorzugte Ausführungsform wird das erfindungsgemäße Verfahren so durchgeführt, dass sich über die Zugabeeinrichtung (11) im Gasstrom eine Temperaturerniedrigung um 0,5 bis 50 K, bevorzugt um 1 bis 40 K und besonders bevorzugt um 3 bis 30 K einstellt.

In einer weiteren bevorzugten Ausführungsform des erfindungsgemäßen Verfahrens weist das Kühlfluid eine Temperatur von ≥ 40 bis ≤ 130 °C, bevorzugt von ≥ 60 bis ≤ 120 °C und besonders bevorzugt von ≥ 80 bis ≤ 110 °C auf. Dieser Temperaturbereich führt zu einer effektiven Reduktion der Ablagerungen und verhindert eine zu starke Auswaschung von Phosgen aus dem Gasstrom. Gemäß einer weiteren bevorzugten Ausführungsform ist das Kühlfluid entsprechend dem in Schritt (i) als Quenchflüssigkeit verwendeten Lösungsmittels ausgewählt.

In einer weiteren bevorzugten Ausführungsform umfasst das erfindungsgemäße Verfahren die weiteren Schritte:
(iv) Auftrennung des Gasstroms aus der ersten Gasleitung (9) in einer zweiten Abtrenneinrichtung (10) in einen Flüssigkeitsstrom und einen Gasstrom und
(v) Rückführung des in Schritt (iv) erhaltenen Flüssigkeitsstroms als Quenchflüssigkeit über eine zweite Flüssigkeitsleitung (13) in die erste Abtrenneinrichtung (2) wobei ein Teilstrom als Kühlfluid in die Kühlfluidzuleitung (12) abgezweigt werden kann, bevorzugt abgezweigt wird.

Hierbei ist es besonders bevorzugt, wenn der in Schritt (v) rückgeführte Flüssigkeitsstrom aus dem Sumpf der zweiten Abtrenneinrichtung (10) entnommen wird. So wird auf besonders wirtschaftliche Weise das in der zweiten Abtrenneinrichtung ausgewaschene Isocyanat in der ersten Abtrenneinrichtung (2) mit dem Rohprodukt vereint und anschließend gemeinsam mit dem darin enthaltenen Isocyanat aufgearbeitet. Über den optionalen eingesetzten Wärmetauscher (16) können die in Schritt (v) rückgeführten Flüssigkeitsströme temperiert werden, was einen zusätzlichen Freiheitsgrad bei der Einstellung der geeigneten Betriebsparameter bietet.

In einer weiteren bevorzugten Ausführungsform des erfindungsgemäßen Verfahrens ist das Kühlfluid Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und/oder Xylol, bevorzugt Chlorbenzol, o-Dichlorbenzol und/oder p-Dichlorbenzol und besonders bevorzugt Chlorbenzol oder o-Dichlorbenzol.

Die unmittelbare Zugabe eines Kühlfluids in einen Gasstrom aus der Quenchzone bei der Herstellung organischer Isocyanate führt zu geringerer Bildung von Ablagerungen und erlaubt daher einen längeren kontinuierlichen Betrieb bevor dieser zu Reinigungszwecken unterbrochen werden muss, was einen direkten positiven Einfluss auf die Raum-Zeit-Ausbeute hat. Somit ist die Verwendung eines Kühlfluids zur Optimierung des Phosgen zu Isocyanat Massenverhältnisses eines Gasstroms aus der ersten Abtrenneinrichtung (2), vorzugsweise aus der Quenchzone, einer Isocyanatherstellung durch unmittelbare Zugabe des Kühlfluids in eine diesen Gasstrom führende erste Gasleitung (9) ein weiterer Gegenstand der vorliegenden Erfindung. Hierbei weist die erste Abtrenneinrichtung (2) wenigstens eine Rohproduktzuleitung (3) für einen mindestens das Isocyanat, Chlorwasserstoff und nicht umgesetztes Phosgen enthaltenden gasförmigen Rohproduktstrom, eine erste Flüssigkeitszuleitung (4) für einen mindestens eine Quenchflüssigkeit enthaltenden Flüssigkeitsstrom und eine erste Flüssigkeitsableitung (5) für einen mindestens einen Teil der Quenchflüssigkeit und einen Teil des Isocyanats enthaltenden Flüssigkeitsstrom und eine erste Gasleitung (9) zur Ableitung eines mindestens Chlorwasserstoff, verdampfte Quenchflüssigkeit und Phosgen enthaltenden Gasstroms auf. Bei dieser Verwendung kommen bevorzugt die vorstehend genannten Kühlfluide, Quenchflüssigkeiten und/oder Lösungsmittel zum Einsatz.

Durch die in der erfindungsgemäßen Reinigungsvorrichtung (1) und/oder im erfindungsgemäßen Verfahren und/oder bei der erfindungsgemäßen Verwendung des Kühlfluids zum Einsatz kommende Zugabeeinrichtung (11) weist der behandelte Gasstrom besondere Eigenschaften auf, die eine nachfolgende Behandlung vereinfachen.

Offenbart ist daher ein Gasstrom zur Behandlung in mindestens einer zweiten Abtrenneinrichtung (10), bevorzugt in mindestens einer Waschkolonne (10), wobei der Gasstrom beim Eintritt in die zweite Abtrenneinrichtung (10) Phosgen und Isocyanat in einem Massenverhältnis von ≥ 1:1 bis ≤ 10000:1, bevorzugt von ≥ 1:1 bis ≤ 5000:1 und besonders bevorzugt von ≥ 1:1 bis ≤ 1000:1 aufweist. Hierbei gelten für die zweite Abtrenneinrichtung (10), bevorzugt die Waschkolonne (10), ebenfalls die vorstehend genannten allgemeinen und bevorzugten Ausgestaltungen. Vorzugsweise entspricht der Gasstrom dem durch die erste Gasleitung (9) führbaren Gasstrom. Sollte der Gasstrom, unabhängig von seiner Herkunft, Flüssigkeitsanteile enthalten, so beziehen sich die vorstehend genannten Massenverhältnisse auf die Gehalte in der Gasphase.

Die vorliegende Erfindung wird mit Bezug auf die nachfolgenden Figuren und Beispiele näher erläutert, ohne jedoch darauf beschränkt zu sein. Es zeigen:
**FIG. 1** schematisch die aus FIG. 2 der WO 2014/122180 A1 bekannte Reinigungsvorrichtung (100).
**FIG. 2** schematisch eine Ausführungsform der erfindungsgemäßen Reinigungsvorrichtung (1).
**FIG. 3A, FIG. 3B, FIG. 3C, FIG. 3D** und **FIG. 3E** schematisch verschiedene Ausführungsformen der Zugabeeinrichtung (11).

In **FIG. 1** ist schematisch eine Ausführungsform einer aus der WO 2014/122180 A1 bekannten Reinigungsvorrichtung (100) zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt dargestellt.

Die Reinigungsvorrichtung (100) besteht aus einer ersten Abtrenneinrichtung (200), die eine Rohproduktzuleitung (300) für einen mindestens das Isocyanat, Chlorwasserstoff und nicht umgesetztes Phosgen enthaltenden gasförmigen Rohproduktstrom aufweist. Weiterhin weist die erste Abtrenneinrichtung (200) eine erste Flüssigkeitsableitung (400) für die Ableitung mindestens einen Teil der Quenchflüssigkeit und einen Teil des Isocyanats enthaltenden Flüssigkeitsstroms auf.

Von der ersten Abtrenneinrichtung (200) geht außerdem eine erste Gasleitung (500) zur Beförderung eines mindestens Chlorwasserstoff, verdampfte Quenchflüssigkeit und Phosgen enthaltenden Gasstroms ab und mündet in einem ersten Kondensator (600). Dieser Kondensator ist so eingerichtet, dass zumindest ein Teil des durch die erste Gasleitung (500) führbaren Gasstroms kondensiert wird. Vom Kondensator (600) geht eine erste Flüssigkeitsleitung (700) zur Beförderung der kondensierten Flüssigkeit ab.

Die Flüssigkeitsleitung (700) mündet in der ersten Abtrenneinrichtung (200). Der Flüssigkeitsleitung (700) ist eine Pumpe (800) zur Zwangsförderung der durch die Flüssigkeitsleitung (700) führbaren Flüssigkeit zugeordnet. Der Kondensator (600) kann zusätzlich noch eine zweite Flüssigkeitsleitung (900) zur Zuleitung eines Teils des durch die erste Flüssigkeitsleitung (700) führbaren Flüssigkeitsstroms und/oder eine dritte Flüssigkeitsleitung (1000) zur Zuleitung eines Lösungsmittelstroms aufweisen. Vom Kondensator geht eine zweite Gasleitung (1100) zur Beförderung des gasförmigen, nicht-kondensierten Anteils ab und mündet in einer zweiten Abtrenneinrichtung (1200).

Von der zweiten Abtrenneinrichtung (1200) geht eine vierte Flüssigkeitsleitung (1300) für die Rückführung eines Flüssigkeitsstroms als Quenchflüssigkeit und mündet in der ersten Abtrenneinrichtung (200). Der vierten Flüssigkeitsleitung (1300) ist eine Pumpe (1400) zur Zwangsförderung des durch die vierte Flüssigkeitsleitung (1300) führbaren Flüssigkeitsstroms zugeordnet.

Die zweite Abtrenneinrichtung (1200) weist eine dritte Gasleitung (1500) zur Ableitung eines nicht-kondensierten Gasstroms auf. Der dritten Gasleitung (1500) ist ein zweiter Kondensator (1600) zur zumindest teilweisen Kondensation des durch dritte Gasleitung (1500) führbaren Gasstroms zugeordnet. Der zweite Kondensator (1600) weist eine vierte Gasleitung (1700) zur Ableitung des nicht-kondensierten Gasstroms auf. Weiterhin weist der zweite Kondensator (1600) eine fünfte Flüssigkeitsleitung (1800) zur Rückführung des kondensierten Flüssigkeitsstroms auf, die in die zweite Abtrenneinrichtung (1200) mündet.

Die zweite Abtrenneinrichtung (1200) kann zusätzlich eine sechste Flüssigkeitsleitung (1900) zur Zugabe eines Flüssigkeitsstroms aufweisen, die im oberen Teil der zweiten Abtrenneinrichtung (1200) angeordnet ist. Weiterhin oder alternativ dazu kann die zweite Abtrenneinrichtung (1200) eine siebte Flüssigkeitsleitung (2000) zur Zugabe eines weiteren Flüssigkeitsstroms aufweisen, die im unteren Teil der zweiten Abtrenneinrichtung (1200) angeordnet ist.

Bei Betrieb der in **FIG. 1** beschriebenen Reinigungsvorrichtung (100) wird zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt zunächst ein mindestens das Isocyanat, Chlorwasserstoff und nicht umgesetztes Phosgen enthaltender gasförmiger Rohproduktstrom über die Rohproduktzuleitung (300) in die erste Abtrenneinrichtung (200) geleitet.

In der ersten Abtrenneinrichtung (200) wird dieser Rohproduktstrom durch Zugabe eines mindestens eine Quenchflüssigkeit enthaltenden Flüssigkeitsstroms über die erste Flüssigkeitsleitung (700) teilweise kondensiert. Die erste Abtrenneinrichtung (200) verlässt ein mindestens einen Teil der Quenchflüssigkeit und einen Teil des Isocyanats enthaltender Flüssigkeitsstrom über die erste Flüssigkeitsableitung (400).

Weiterhin verlässt die erste Abtrenneinrichtung (200) ein mindestens Chlorwasserstoff, verdampfte Quenchflüssigkeit und Phosgen enthaltender Gasstrom über die erste Gasleitung (500) zum ersten Kondensator (600) hin. Dieser Gasstrom wird im Kondensator (600) zumindest teilweise kondensiert. Die dabei erhaltene Flüssigkeit wird durch die erste Flüssigkeitsleitung (700) mittels der Pumpe (800) zwangsgefördert in die erste Abtrenneinrichtung (200) zurückgeführt.

Ein Teil der durch die erste Flüssigkeitsleitung (700) geführten Flüssigkeit kann in die zweite Flüssigkeitsleitung (900) abgezweigt werden und so dem Kondensator (600) wieder zugeleitet werden. Alternativ oder zusätzlich kann weiteres Lösungsmittel über die dritte Flüssigkeitsleitung (1000) zugeleitet werden. Der im Kondensator (600) gasförmige, nicht-kondensierte Anteil wird durch die zweite Gasleitung (1100) in die zweite Abtrenneinrichtung (1200) befördert.

Die in der zweiten Abtrenneinrichtung (1200) erhaltene Flüssigkeit wird über die vierte Flüssigkeitsleitung (1300) als Quenchflüssigkeit in die erste Abtrenneinrichtung (200) zurückgeführt. Diese Zurückführung erfolgt zwangsgefördert durch die Pumpe (1400). Der in der der zweiten Abtrenneinrichtung (1200) nicht-kondensierte Gasstrom wird über die dritte Gasleitung (1500) abgeleitet und im zweiten Kondensator (1600) zumindest teilweise kondensiert. Der hierbei nicht-kondensierte Anteil wird aus dem Kondensator (1600) über die vierte Gasleitung (1700) abgeleitet. Über die Flüssigkeitsleitung (1800) wird der verflüssigte Anteil in die zweite Abtrenneinrichtung (1200) zurückgeführt.

Über die im oberen Teil der zweiten Abtrenneinrichtung (1200) angeordnete, optionale, sechste Flüssigkeitsleitung (1900) kann der zweiten Abtrenneinrichtung (1200) ein zusätzlicher Flüssigkeitsstrom zugeführt werden. Ein weiterer Flüssigkeitsstrom kann der zweiten Abtrenneinrichtung (1200) über die im unteren Teil der zweiten Abtrenneinrichtung (1200) angeordnete, optionale, siebte Flüssigkeitsleitung (2000) zugeführt werden.

In **FIG. 2** ist eine schematische Ausführungsform der erfindungsgemäßen Reinigungsvorrichtung (1) zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt dargestellt.

Die Reinigungsvorrichtung (1) besteht aus einer ersten Abtrenneinrichtung (2), die eine Rohproduktzuleitung (3) für einen mindestens das Isocyanat, Chlorwasserstoff und nicht umgesetztes Phosgen enthaltenden gasförmigen Rohproduktstrom aufweist. In die Abtrenneinrichtung (2) mündet eine erste Flüssigkeitszuleitung (4) für einen mindestens eine Quenchflüssigkeit enthaltenden Flüssigkeitsstrom. Weiterhin weist die erste Abtrenneinrichtung (2) eine erste Flüssigkeitsableitung (5) für einen mindestens einen Teil der Quenchflüssigkeit und einen Teil des Isocyanats enthaltenden Flüssigkeitsstrom auf. Von der ersten Flüssigkeitsableitung (5) geht eine erste Flüssigkeitsleitung (6) für die Rückförderung eines Teils des durch die erste Flüssigkeitsableitung (5) führbaren Flüssigkeitsstroms ab und mündet in die erste Flüssigkeitszuleitung (4). Der ersten Flüssigkeitsleitung (6) ist eine Pumpe (7) zur Zwangsförderung dieses Flüssigkeitsstroms und ein Quenchekühler (8) zur Kühlung des durch die erste Flüssigkeitsleitung führbaren Flüssigkeitsstroms zugeordnet.

Von der ersten Abtrenneinrichtung (2) geht außerdem eine erste Gasleitung (9) zur Beförderung eines mindestens Chlorwasserstoff, verdampfter Quenchflüssigkeit und Phosgen enthaltenden Gasstroms ab und führt zu einer zweiten Abtrenneinrichtung (10). Der ersten Gasleitung (9) ist eine Zugabeeinrichtung (11) zum unmittelbaren Einbringen mindestens eines Kühlfluids zugeordnet. Die Zugabeeinrichtung (11) weist eine Kühlfluidzuleitung (12) zur Beförderung des Kühlfluids auf.

Von der zweiten Abtrenneinrichtung (10) geht eine zweite Flüssigkeitsleitung (13) für die teilweise Rückführung eines Flüssigkeitsstroms als Quenchflüssigkeit ab und mündet in der ersten Abtrenneinrichtung (2). Die Kühlfluidzuleitung (12) kann unter anderem direkt einem Vorlagebehälter für das Kühlfluid entspringen. Bevorzugt entspringt sie als Abzweig aus der zweiten Flüssigkeitsleitung (13), so dass beide Leitungen (12 und 13) über eine, der zweiten Flüssigkeitsleitung (13) zugeordnete Pumpe (14) versorgt werden können. Der zweiten Flüssigkeitsleitung (13) und der Kühlfluidzuleitung (12) sind weiterhin Regelungseinrichtungen (15a) und (15b) zur Steuerung der Flüssigkeitsmengenströme durch die zweite Flüssigkeitsleitung (13) zur ersten Abtrenneinrichtung (2) einerseits und durch die Kühlfluidzuleitung (12) zur Zugabeeinrichtung (11) andererseits zugeordnet. Stromabwärts nach der Pumpe (14) ist der zweiten Flüssigkeitsleitung (13) zusätzlich ein Wärmetauscher (16) zur Regulierung der Temperatur des durch die zweite Flüssigkeitsleitung (13) führbaren Flüssigkeitsstroms zugeordnet.

Die zweite Abtrenneinrichtung (10) weist eine zweite Gasleitung (17) zur Ableitung eines nicht-kondensierten Gasstroms auf. Der zweiten Gasleitung (17) ist ein Kondensator (18) zur zumindest teilweisen Kondensation des durch zweite Gasleitung (17) führbaren Gasstroms zugeordnet. Der Kondensator (18) weist eine dritte Gasleitung (19) zur Ableitung des nicht-kondensierten Gasstroms auf. Weiterhin weist der Kondensator (18) eine dritte Flüssigkeitsleitung (20) zur Rückführung des kondensierten Flüssigkeitsstroms auf, die in die zweite Abtrenneinrichtung (10) mündet.

Die zweite Abtrenneinrichtung (10) weist zusätzlich eine vierte Flüssigkeitsleitung (21) zur Zugabe eines Flüssigkeitsstroms auf, die im oberen Teil der zweiten Abtrenneinrichtung (10) angeordnet ist. Weiterhin kann die zweite Abtrenneinrichtung (10) eine fünfte Flüssigkeitsleitung (22A) zur Zugabe eines weiteren Flüssigkeitsstroms aufweisen, die im unteren Teil der zweiten Abtrenneinrichtung (10) angeordnet ist. Alternativ kann diese fünfte Flüssigkeitsleitung (22A) als fünfte Flüssigkeitsleitung (22B) in die zweite Flüssigkeitsleitung (13) münden.

Bei Betrieb der in **FIG. 2** beschriebenen Reinigungsvorrichtung (1) wird zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt zunächst ein mindestens das Isocyanat, Chlorwasserstoff und nicht umgesetztes Phosgen enthaltender gasförmiger Rohproduktstrom über die Rohproduktzuleitung (3) in die erste Abtrenneinrichtung (2) geleitet.

In der ersten Abtrenneinrichtung (2) wird dieser Rohproduktstrom durch Zugabe eines mindestens eine Quenchflüssigkeit enthaltenden Flüssigkeitsstroms über die erste Flüssigkeitszuleitung (4) teilweise kondensiert. Die erste Abtrenneinrichtung (2) verlässt ein mindestens einen Teil der Quenchflüssigkeit und einen Teil des Isocyanats enthaltender Flüssigkeitsstrom über die erste Flüssigkeitsableitung (5). Ein Teil dieses Flüssigkeitsstroms wird über die erste Flüssigkeitsleitung (6) mittels der Pumpe (7) zwangsgefördert und mittels des Quenchekühlers (8) gekühlt in die erste Flüssigkeitszuleitung (4) rückgeführt und so wieder als Quenchflüssigkeit eingesetzt.

Weiterhin verlässt die Abtrenneinrichtung (2) ein mindestens Chlorwasserstoff, verdampfte Quenchflüssigkeit und Phosgen enthaltender Gasstrom über die erste Gasleitung (9) zu der zweiten Abtrenneinrichtung (10). In die erste Gasleitung (9) wird mindestens ein gegenüber Isocyanatgruppen inerten Kühlfluids über die Zugabeeinrichtung (11) unmittelbar eingebracht, wodurch der Gasstrom wenigstens teilweise kondensiert und/oder absorbiert wird. Das Kühlfluid wird über die Kühlfluidzuleitung (12) zur Zugabeeinrichtung (11) befördert.

Der teilweise kondensierte und/oder absorbierte Gasstrom aus der ersten Gasleitung (9) wird in der zweiten Abtrenneinrichtung (10) weiter behandelt. Bei dieser Behandlung wird ein Flüssigkeitsstrom erhalten, der zumindest teilweise über die zweite Flüssigkeitsleitung (13) als Quenchflüssigkeit zur ersten Abtrenneinrichtung (2) rückgeführt wird. Die Rückführung erfolgt zwangsgefördert über die, der zweiten Flüssigkeitsleitung (13) zugeordnete Pumpe (14). Die Kühlfluidzuleitung (12) kann unter anderem direkt einem Vorlagebehälter für das Kühlfluid entspringen. Bevorzugt entspringt sie als Abzweig aus der zweiten Flüssigkeitsleitung (13), so dass beide Leitungen (12 und 13) über die Pumpe (14) versorgt werden.

Die Flüssigkeitsmengenströme durch die zweite Flüssigkeitsleitung (13) zur ersten Abtrenneinrichtung (2) einerseits und durch die Kühlfluidzuleitung (12) zur Zugabeeinrichtung (11) andererseits werden durch die Regelungseinrichtungen (15a) und (15b) gesteuert. Die Temperatur der Flüssigkeitsströme wird durch den Wärmetauscher (16) reguliert.

Der in der zweiten Abtrenneinrichtung (10) nicht-kondensierte Anteil wird über die zweite Gasleitung (17) abgeleitet und mittels des, der zweiten Gasleitung (17) zugeordneten Kondensators (18) zumindest teilweise kondensiert. Der hierbei gasförmig bleibende Anteil wird als nichtkondensierter Gasstrom über die dritte Gasleitung (19) aus dem Kondensator abgeleitet. Der verflüssigte Anteil wird aus dem Kondensator (18) über die dritte Flüssigkeitsleitung (20) in die zweite Abtrenneinrichtung (10) rückgeführt. Über die vierte Flüssigkeitsleitung (21) und/oder die fünfte Flüssigkeitsleitung (22A) oder (22B) kann im Bedarfsfall zusätzliche Flüssigkeit in die die zweite Abtrenneinrichtung (10) gegeben werden.

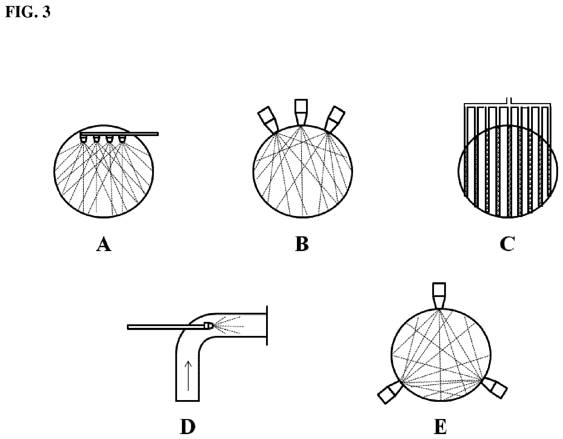

In **FIG. 3** sind verschiedene Ausführungsformen der Zugabeeinrichtung (11) schematisch dargestellt.

So zeigt **FIG. 3A** einen Querschnitt durch die erste Gasleitung (9) wobei das Kühlfluid über eine an ihrer Unterseite mit Düsen bestückte Lanze in die Gasleitung eingebracht wird.

**FIG. 3B** zeigt eine Anordnung, bei der die Düsen in die Wandung der ersten Gasleitung (9) integriert sind.

In **FIG. 3C** ist eine Variante der Zugabeeinrichtung ohne die Verwendung von Düsen dargestellt. Das Kühlfluid wird hier durch einfache Bohrungen im oberen Bereich der Wandung der ersten Gasleitung (9) zugegeben. Es bildet sich ein lamellenartiger Vorhang aus, der das durch die Gasleitung strömende Gemisch kreuzt.

**FIG. 3D** stellt eine weitere Variante der Zugabeeinrichtung dar. Hierbei handelt es sich um einen Längsschnitt durch die erste Gasleitung (9) wobei das Kühlfluid über eine Lanze zugegeben wird, an deren Ende sich eine Düse zum feinen Versprühen des Kühlfluids befindet. Die Gasleitung enthält dabei einen 90° Rohrbogen und die Lanze ist so angeordnet, dass sich die Düse stromabwärts des Rohrbogens befindet und das Kühlfluid im Gleichstrom mit dem durch die Gasleitung strömenden Stoffgemisch versprüht.

In **FIG. 3E** ist wiederum eine Anordnung im Querschnitt dargestellt, bei der die Düsen in die Wandung der ersten Gasleitung (9) integriert sind, wobei sie diesmal gleichmäßig über den Rohrumfang verteilt sich nicht wie in FIG. 3B alle auf der oberen Hälfte des Rohrquerschnitts befinden.

### Beispiele

### Beispiel 1 (Vergleichsbeispiel):

In einer Versuchsanlage zur Herstellung von Hexamethylendiisocyanat in der Gasphase wurde der Reaktionszone eine erste Abtrenneinrichtung nachgeschaltet, in der das Rohprodukt mittels Zugabe von Chlorbenzol abgekühlt wurde. Dabei wurden 99% des Isocyanats kondensiert und in Chlorbenzol gelöst als flüssiges Rohprodukt abgetrennt. Der nicht kondensierte Anteil des die Reaktionszone verlassenden Rohprodukts wurde in einem Rohrbündelwärmetauscher um weitere 10 K auf 120 °C abgekühlt. Der Wärmetauscher wurde dabei mit gerade so viel Chlorbenzol berieselt, dass seine Wärmeübertragungsflächen gleichmäßig benetzt waren, um Anbackungen zu vermeiden. Der Gasstrom befand sich dabei auf der Rohrseite des Kondensators und die Wärmeabfuhr erfolgte über einen Kühlwasserstrom in der Mantelseite des Kondensators. Zur Temperaturregelung konnte der Kühlwasserstrom mittels eines Kugel-Ventils angedrosselt werden. Außerdem wurde die gasseitige Ein- und Austrittstemperatur kontinuierlich über den Versuchszeitraum gemessen. Im weiteren Verlauf wurde der so abgekühlte Rohproduktstrom in eine Waschkolonne geleitet, an deren Kopf ein Strom aus Chlorbenzol und Phosgen entnommen wurde während organische Verunreinigungen am Sumpf aufkonzentriert und als flüssiger Strom ausgeschleust wurden. In dieser Waschkolonne wurde die Druckdifferenz zwischen dem Gasraum unterhalb der untersten Trennstufe und dem Gasraum oberhalb der obersten Trennstufe kontinuierlich gemessen.

In thermodynamischen Modellrechnungen (Aspen Plus® V7.2) konnte gezeigt werden, dass ca. 0,1% des in den Kondensator eintretenden Phosgens im Kondensator als Flüssigkeit niedergeschlagen wurden. Hierfür wurde ein gasförmiger Strom bestehend aus 20 Gew.-% Phosgen, 15 Gew.-% HCl und 65 Gew.-% Chlorbenzol bei 1,5 bar(a) in einem "Flash2" von 130 °C auf 120 °C abgekühlt und die Verteilung des Phosgens auf den flüssigen und den gasförmigen Auslassstrom des Flashs untersucht.

Innerhalb von ca. 12 Tagen kontinuierlichen Betriebs stieg der Druckverlust in der Waschkolonne von ursprünglich 102 mbar auf einen unzulässig hohen Wert von 150 mbar an.

### Beispiel 2 (Vergleichsbeispiel):

Es wurde mit der gleichen Anordnung wie im Beispiel 1 gearbeitet. Diesmal wurde jedoch die prozessseitige Austrittstemperatur des Kondensators auf 50 °C eingestellt. Zwar konnte mit dieser Kondensationstemperatur die Waschkolonne wesentlich länger betrieben werden als in Beispiel 1, doch es kam zu einer starken Anreicherung von Phosgen im anfallenden Kondensat.

In thermodynamischen Modellrechnungen (Aspen Plus® V7.2) konnte gezeigt werden, dass ca. 20% des in den Kondensator eintretenden Phosgens im Kondensator als Flüssigkeit niedergeschlagen wurden. Hierfür wurde ein gasförmiger Strom bestehend aus 20 Gew.-% Phosgen, 15 Gew.-% HCl und 65 Gew.-% Chlorbenzol bei 1,5 bar(a) in einem "Flash2" von 130 °C auf 50 °C abgekühlt und die Verteilung des Phosgens auf den flüssigen und den gasförmigen Auslassstrom des Flashs untersucht.

### Beispiel 3 (erfindungsgemäß):

Die Anordnung aus Beispiel 1 wurde dahingehend modifiziert, dass der Rohrbündelwärmetauscher samt erster Flüssigkeitsableitung durch ein einfaches Rohrstück ersetzt wurde, das mit einer Zugabeeinrichtung für Chlorbenzol ausgestattet war. Dabei handelte es sich um eine einfache Einstoff-Düse, die sich wie in FIG. 3D skizziert mittig in der Rohrleitung befand und über die das Chlorbenzol im Gleichstrom zentral in die Rohrleitung versprüht werden konnte. Der Druck in der Chlorbenzol-Zuleitung zur Düse wurde so eingestellt, dass der Druckverlust beim Austritt der Düse zu einer feinen Zerstäubung der Flüssigkeit führte. Die Düse erzeugte im Gleichstrom mit dem Gasstrom einen Vollkegel mit 45° Öffnungswinkel. Der Massenstrom an Chlorbenzol zur Zugabeeinrichtung wurde so gewählt, dass das Verhältnis aus diesem und dem Massenstrom an nicht kondensiertem Rohprodukt zur Zugabeeinrichtung 1:7 betrug, die Temperatur des Chlorbenzols im Zulauf der Zugabeeinrichtung betrug 95 °C, der Gasstrom hatte stromaufwärts von der Zugabeeinrichtung eine Temperatur von 130 °C. Es stellte sich stromabwärts der Zugabeeinrichtung eine Temperatur von 122 °C ein. Nach 27 Tagen wurde der Versuch abgebrochen. Der Druckverlust über die Zugabeeinrichtung sowie über die Waschkolonne blieb in diesem Zeitraum konstant. Es zeigten sich auch bei der anschließenden Inspektion der Apparate keine Anzeichen von Ablagerungen.

In thermodynamischen Modellrechnungen (Aspen Plus® V7.2) konnte gezeigt werden, dass ca. 0,5% des durch die Gasleitung geführten Phosgens in der Flüssigkeit niedergeschlagen wurden. Hierfür wurde ein gasförmiger, 130 °C warmer Strom bestehend aus 20 Gew.-% Phosgen, 15 Gew.-% HCl und 65 Gew.-% Chlorbenzol in einem "Mixer" mit einem flüssigen, 95°C warmen Chlorbenzol-Strom vermischt. Das Gewichtsverhältnis wurde analog zum Versuch auf 1:7 (Chlorbenzol:Gasstrom) gesetzt. Der gemischte Strom wurde dann bei 1,5 bar(a) ohne weitere Wärmezu- oder abfuhr in einem "Flash2" in einen gasförmigen und einen flüssigen Strom getrennt und die Verteilung des Phosgens auf diese beiden Ströme untersucht.

### Beispiel 4 (erfindungsgemäß):

In einer Versuchsanlage zur Herstellung von Isophorondiisocyanat in der Gasphase wurde der Reaktionszone eine erste Abtrenneinrichtung nachgeschaltet, in der das Rohprodukt mittels Zugabe von Chlorbenzol abgekühlt wurde. Dabei wurden 99,2% des Isocyanats kondensiert und in Chlorbenzol gelöst als flüssiges Rohprodukt abgetrennt. Der nicht kondensierte Anteil des die Reaktionszone verlassenden Rohprodukts hatte eine Temperatur von 145 °C und wurde mittels einer Rohrleitung aus dem Gas-Flüssig-Abscheider abgeleitet. In dieser Rohrleitung befand sich eine Zugabeeinrichtung für Chlorbenzol entsprechend der Anordnung in Beispiel 3. Abweichend von Beispiel 3 wurde der Massenstrom an Chlorbenzol zur Zugabeeinrichtung diesmal so gewählt, dass das Verhältnis aus diesem und dem Massenstrom an nicht kondensiertem Rohprodukt zur Zugabeeinrichtung 1:3 betrug. Es stellte sich stromabwärts der Zugabeeinrichtung eine Temperatur von 122 °C ein. Nach 25 Tagen wurde der Versuch abgebrochen. Der Druckverlust über die Zugabeeinrichtung sowie über die Waschkolonne blieb in diesem Zeitraum konstant. Es zeigten sich auch bei der anschließenden Inspektion der Apparate keine Anzeichen von Ablagerungen.

In thermodynamischen Modellrechnungen (Aspen Plus® V7.2) konnte gezeigt werden, dass ca. 1% des durch die Gasleitung geführten Phosgens in der Flüssigkeit niedergeschlagen wurden. Hierfür wurde ein gasförmiger, 145 °C warmer Strom bestehend aus 20 Gew.-% Phosgen, 15 Gew.-% HCl und 65 Gew.-% Chlorbenzol in einem "Mixer" mit einem flüssigen, 95°C warmen Chlorbenzol-Strom vermischt. Das Gewichtsverhältnis wurde analog zum Versuch auf 1:3 (Chlorbenzol:Gasstrom) gesetzt. Der gemischte Strom wurde dann bei 1,5 bar(a) ohne weitere Wärmezu- oder abfuhr in einem "Flash2" in einen gasförmigen und einen flüssigen Strom getrennt und die Verteilung des Phosgens auf diese beiden Ströme untersucht.

### Beispiel 5 (erfindungsgemäß):

In einer Versuchsanlage zur Herstellung von Toluylendiisocyanat in der Gasphase wurde der Reaktionszone eine erste Abtrenneinrichtung nachgeschaltet, in der das Rohprodukt mittels Zugabe von o-Dichlorbenzol abgekühlt wurde. Dabei wurden 98,5 % des Isocyanats kondensiert und in o-Dichlorbenzol gelöst als flüssiges Rohprodukt abgetrennt. Der nicht kondensierte Anteil des die Reaktionszone verlassenden Rohprodukts hatte eine Temperatur von 170 °C und wurde mittels einer Rohrleitung aus dem Gas-Flüssig-Abscheider abgeleitet. In dieser Rohrleitung befand sich eine Zugabeeinrichtung für Kühlfluid. Anders als in Beispiel 3 wurde diesmal das Kühlfluid am Sumpf der zweiten Abtrenneinrichtung entnommen und mittels eines Rohrbündelwärmetauschers auf 85 °C temperiert. Der Massenstrom des Kühfluids wurde so gewählt, dass das Verhältnis aus diesem und dem Massenstrom an nicht kondensiertem Rohprodukt zur Zugabeeinrichtung 1:2 betrug. Es stellte sich stromabwärts der Zugabeeinrichtung eine Temperatur von 157 °C ein. Nach 22 Tagen wurde der Versuch abgebrochen. Der Druckverlust über die Zugabeeinrichtung sowie über die Waschkolonne war in diesem Zeitraum von 108 mbar auf 120 mbar angestiegen.

In thermodynamischen Modellrechnungen (Aspen Plus® V7.2) konnte gezeigt werden, dass ca. 1,6% des durch die Gasleitung geführten Phosgens in der Flüssigkeit niedergeschlagen wurden. Hierfür wurde ein gasförmiger, 170 °C warmer Strom bestehend aus 20 Gew.-% Phosgen, 15 Gew.-% HCl und 65 Gew.-% o-Dichlorbenzol in einem "Mixer" mit einem flüssigen, 85°C warmen Strom aus 97,5 Gew.-% o-Dichlorbenzol und 2,5 Gew.-% Toluylendiisocyanat vermischt. Das Gewichtsverhältnis wurde analog zum Versuch auf 1:2 (o-Dichlorbenzol:Gasstrom) gesetzt. Die gerechnete Temperatur der Mischung betrug wie im Versuch 157 °C. Der gemischte Strom wurde dann bei 1,5 bar(a) ohne weitere Wärmezu- oder abfuhr in einem "Flash2" in einen gasförmigen und einen flüssigen Strom getrennt und die Verteilung des Phosgens auf diese beiden Ströme untersucht.

## Patentansprüche

1. Reinigungsvorrichtung (1) zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt, umfassend eine erste Abtrenneinrichtung (2), die wenigstens
- eine Rohproduktzuleitung (3) für einen mindestens das Isocyanat, Chlorwasserstoff und nicht umgesetztes Phosgen enthaltenden gasförmigen Rohproduktstrom,
- eine erste Flüssigkeitszuleitung (4) für einen mindestens eine Quenchflüssigkeit enthaltenden Flüssigkeitsstrom und
- eine erste Flüssigkeitsableitung (5) für einen mindestens einen Teil der Quenchflüssigkeit und einen Teil des Isocyanats enthaltenden Flüssigkeitsstrom aufweist,
wobei von der ersten Abtrenneinrichtung (2) eine erste Gasleitung (9) zur Beförderung eines mindestens Chlorwasserstoff, verdampfte Quenchflüssigkeit und Phosgen enthaltenden Gasstroms abgeht,
**dadurch gekennzeichnet, dass** der ersten Gasleitung (9) mindestens eine Zugabeeinrichtung (11) zum unmittelbaren Einbringen mindestens eines Kühlfluids für eine wenigstens teilweise Kondensation und/oder Absorption des durch die erste Gasleitung (9) führbaren Gasstroms zugeordnet ist.

2. Reinigungsvorrichtung (1) nach Anspruch 1, **dadurch gekennzeichnet, dass** die erste Abtrenneinrichtung (2) und/oder die erste Gasleitung (9) so eingerichtet sind, dass der durch die erste Gasleitung (9) führbare Gasstrom vor dem Erreichen der Zugabeeinrichtung (11) mindestens einmal, bevorzugt mindestens zweimal und besonders bevorzugt zwei bis fünfmal umgelenkt wird.

3. Reinigungsvorrichtung (1) nach Anspruch 1 oder 2, **dadurch gekennzeichnet, dass** die Zugabeeinrichtung (11) aus einer oder mehreren Düsen zum unmittelbaren Einbringen des Kühlfluids im Gleich- oder Gegenstrom, bevorzugt im Gleichstrom, besteht oder aus mehreren Düsen besteht, die im Leitungsmantel der ersten Gasleitung (9), bevorzugt in gleichmäßigen Abständen entlang des Umfangs der ersten Gasleitung (9), positioniert sind.

4. Reinigungsvorrichtung (1) nach Anspruch 1 oder 2, **dadurch gekennzeichnet, dass** die Zugabeeinrichtung (11) mindestens eine, bevorzugt mehrere Öffnungen zum Erzeugen eines Fluidvorhangs über den gesamten Querschnitt der ersten Gasleitung (9) aufweist, die in einem horizontal verlaufenden Rohrleitungsteil im oberen Bereich des Leitungsmantels der ersten Gasleitung (9) positioniert ist, so dass der Gasstrom die Lamellen oder Strahlen des Fluidvorhangs kreuzt.

5. Reinigungsvorrichtung (1) nach einem der Ansprüche 1 bis 4, **dadurch gekennzeichnet, dass** die Zugabeeinrichtung (11) eine Kühlfluidzuleitung (12) zur Beförderung des Kühlfluids aufweist.

6. Reinigungsvorrichtung (1) nach einem der Ansprüche 1 bis 5, **dadurch gekennzeichnet, dass** die erste Gasleitung (9) zu einer zweiten Abtrenneinrichtung (10) führt.

7. Reinigungsvorrichtung (1) nach Anspruch 6, **dadurch gekennzeichnet, dass** von der zweiten Abtrenneinrichtung (10) eine zweite Flüssigkeitsleitung (13) zur zumindest teilweisen Rückführung eines Flüssigkeitsstroms als Quenchflüssigkeit abgeht und zur ersten Abtrenneinrichtung (2) hinführt.

8. Reinigungsvorrichtung (1) nach Anspruch 7, **dadurch gekennzeichnet, dass** der zweiten Flüssigkeitsleitung (13) und der Kühlfluidzuleitung (12) zusätzlich Regelungseinrichtungen (15a) und (15b) zur Steuerung der durch die zweite Flüssigkeitsleitung (13) zur ersten Abtrenneinrichtung (2) einerseits und durch die Kühlfluidzuleitung (12) zur Zugabeeinrichtung (11) andererseits führbaren Flüssigkeitsmengenströme zugeordnet sind.

9. Verfahren zur Abtrennung eines durch Umsetzung eines organischen Amins mit einem stöchiometrischen Überschuss an Phosgen in der Gasphase hergestellten organischen Isocyanats aus dem in der Umsetzung erhaltenen gasförmigen Rohprodukt, umfassend die Schritte,
(i) mindestens teilweise Kondensation des Rohproduktstroms, der mindestens das Isocyanat, Chlorwasserstoff und nicht umgesetztes Phosgen enthält, durch Inkontaktbringen mit mindestens einem Flüssigkeitsstrom, der mindestens eine Quenchflüssigkeit enthält, in einer ersten Abtrenneinrichtung (2) unter Erhalt
eines mindestens einen Teil der Quenchflüssigkeit und einen Teil des Isocyanats enthaltenden Flüssigkeitsstroms und
eines mindestens Chlorwasserstoff, verdampfte Quenchflüssigkeit und Phosgen enthaltenden Gasstroms,
(ii) Ableitung des in Schritt (i) erhaltenen Flüssigkeitsstroms über eine erste Flüssigkeitsableitung (5) und des in Schritt (i) erhaltenen Gasstroms über eine erste Gasleitung (9) und
(iii) wenigstens teilweise Kondensation und/oder Absorption des in Schritt (ii) durch die erste Gasleitung (9) abgeleiteten Gasstroms,
**dadurch gekennzeichnet, dass** die wenigstens teilweise Kondensation und/oder Absorption in Schritt (iii) durch unmittelbares Einbringen mindestens eines Kühlfluids erfolgt, wobei das Kühlfluid über mindestens eine Zugabeeinrichtung (11), die der ersten Gasleitung (9) zugeordnet ist, unmittelbar in die erste Gasleitung (9) eingebracht wird.

10. Verfahren nach Anspruch 9, **dadurch gekennzeichnet, dass** das Gewichts-Verhältnis des eingebrachten Kühlfluids zum durch die erste Gasleitung (9) geführten Gasstrom 1:100 bis 10:1, bevorzugt 1:12 bis 1:1 und besonders bevorzugt 1:10 bis 1:2 beträgt.

11. Verfahren nach Anspruch 9 oder 10, **dadurch gekennzeichnet, dass** das Kühlfluid entsprechend dem in Schritt (i) als Quenchflüssigkeit verwendeten Lösungsmittel ausgewählt wird.

12. Verfahren nach einem der Ansprüche 9 bis 11, **dadurch gekennzeichnet, dass** es die weiteren Schritte
(iv) Auftrennung des Gasstroms aus der ersten Gasleitung (9) in einer zweiten Abtrenneinrichtung (10) in einen Flüssigkeitsstrom und einen Gasstrom und
(v) Rückführung des in Schritt (iv) erhaltenen Flüssigkeitsstroms als Quenchflüssigkeit über eine zweite Flüssigkeitsleitung (13) in die erste Abtrenneinrichtung (2) wobei ein Teilstrom als Kühlfluid in die Kühlfluidzuleitung (12) abgezweigt werden kann, bevorzugt abgezweigt wird, umfasst.

13. Verfahren nach einem der Ansprüche 9 bis 12, **dadurch gekennzeichnet, dass** das Kühlfluid Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und/oder Xylol, bevorzugt Chlorbenzol, o-Dichlorbenzol und/oder p-Dichlorbenzol und besonders bevorzugt Chlorbenzol oder o-Dichlorbenzol ist.

14. Verfahren nach einem der Ansprüche 9 bis 13, **dadurch gekennzeichnet, dass** sich über die Zugabeeinrichtung (11) im Gasstrom eine Temperaturerniedrigung um 0,5 bis 50 K, bevorzugt um 1 bis 40 K und besonders bevorzugt um 3 bis 30 K einstellt.

## Claims

1. Cleaning apparatus (1) for separating an organic isocyanate prepared by reacting an organic amine with a stoichiometric excess of phosgene in the gas phase from the gaseous crude product obtained in the reaction, comprising a first separation unit (2) having at least
- a crude product inlet (3) for a gaseous crude product stream containing at least the isocyanate, hydrogen chloride and unconverted phosgene,
- a first liquid inlet (4) for a liquid stream containing at least a quench liquid and
- a first liquid outlet (5) for a liquid stream containing at least some of the quench liquid and some of the isocyanate,
wherein a first gas conduit (9) for conveying a gas stream containing at least hydrogen chloride, evaporated quench liquid and phosgene departs from the first separation unit (2),
**characterized in that** at least one addition unit (11) for direct introduction of at least one cooling fluid for an at least partial condensation and/or absorption of the gas stream conductable through the first gas conduit (9) is assigned to the first gas conduit (9).

2. Cleaning apparatus (1) according to Claim 1, **characterized in that** the first separation unit (2) and/or the first gas conduit (9) are set up such that the gas stream conductable through the first gas conduit (9) is deflected at least once, preferably at least twice and more preferably 2 to 5 times before it reaches the addition unit (11).

3. Cleaning apparatus (1) according to Claim 1 or 2, **characterized in that** the addition unit (11) consists of one or more nozzles for direct introduction of the cooling fluid in cocurrent or countercurrent, preferably in cocurrent, or consists of multiple nozzles positioned in the conduit shell of the first gas conduit (9), preferably at uniform intervals along the circumference of the first gas conduit (9).

4. Cleaning apparatus (1) according to Claim 1 or 2, **characterized in that** the addition unit (11) has at least one orifice and preferably two or more orifices for generation of a fluid curtain over the entire cross section of the first gas conduit (9), positioned in a horizontal pipeline section in the upper region of the conduit shell of the first gas conduit (9), such that the gas flow crosses the lamellae or jets of the fluid curtain.

5. Cleaning apparatus (1) according to any of Claims 1 to 4, **characterized in that** the addition unit (11) has a cooling fluid inlet (12) for conveying the cooling fluid.

6. Cleaning apparatus (1) according to any of Claims 1 to 5, **characterized in that** the first gas conduit (9) leads to a second separation unit (10).

7. Cleaning apparatus (1) according to Claim 6, **characterized in that** a second liquid conduit (13) for at least partial recycling of a liquid stream as quench liquid departs from the second separation unit (10) and leads to the first separation unit (2) .

8. Cleaning apparatus (1) according to Claim 7, **characterized in that** closed-loop control devices (15a) and (15b) for control of the liquid flow rates conductable through the second liquid conduit (13) to the first separation unit (2) on the one hand and through the cooling fluid inlet (12) to the addition unit (11) on the other hand are additionally assigned to the second liquid conduit (13) and to the cooling fluid inlet (12).

9. Process for separating an organic isocyanate prepared by reacting an organic amine with a stoichiometric excess of phosgene in the gas phase from the gaseous crude product obtained in the reaction, comprising the steps of
(i) at least partial condensation of the crude product stream containing at least the isocyanate, hydrogen chloride and unconverted phosgene by contacting with at least one liquid stream containing at least one quench liquid in a first separation unit (2) to obtain
a liquid stream containing at least some of the quench liquid and some of the isocyanate and
a gas stream containing at least hydrogen chloride, evaporated quench liquid and phosgene,
(ii) discharge of the liquid stream obtained in step (i) via a first liquid outlet (5) and of the gas stream obtained in step (i) via a first gas conduit (9) and
(iii) at least partial condensation and/or absorption of the gas stream discharged in step (ii) through the first gas conduit (9), **characterized in that** the at least partial condensation and/or absorption is effected in step (iii) by direct introduction of at least one cooling fluid, wherein the cooling fluid is introduced directly into the first gas conduit (9) via at least one addition unit (11) assigned to the first gas conduit (9).

10. Process according to Claim 9, **characterized in that** the weight ratio of the cooling fluid introduced to the gas stream conducted through the first gas conduit (9) is 1:100 to 10:1, preferably 1:12 to 1:1 and more preferably 1:10 to 1:2.

11. Process according to Claim 9 or 10, **characterized in that** the cooling fluid is selected to correspond to the solvent used as quench liquid in step (i).

12. Process according to any of Claims 9 to 11, **characterized in that** it comprises the further steps of
(iv) separating the gas stream from the first gas conduit (9) in a second separation unit (10) into a liquid stream and a gas stream and
(v) recycling the liquid stream obtained in step (iv) as quench liquid via a second liquid conduit (13) into the first separation unit (2), it being possible and preferable to branch off a substream as cooling fluid into the cooling fluid inlet (12).

13. Process according to any of Claims 9 to 12, **characterized in that** the cooling fluid is chlorobenzene, o-dichlorobenzene, p-dichlorobenzene, trichlorobenzenes, the corresponding chlorotoluenes or chloroxylenes, chloroethylbenzene, monochlorodiphenyl, α- or β-naphthyl chloride, ethyl benzoate, dialkyl phthalates, diisodiethyl phthalate, toluene and/or xylene, preferably chlorobenzene, o-dichlorobenzene and/or p-dichlorobenzene and more preferably chlorobenzene or o-dichlorobenzene.

14. Process according to any of Claims 9 to 13, **characterized in that** the addition unit (11) establishes a lowering of the temperature in the gas stream by 0.5 to 50 K, preferably by 1 to 40 K and more preferably by 3 to 30 K.

## Revendications

1. Dispositif de nettoyage (1) pour séparer un isocyanate organique préparé en phase gazeuse par transformation d'une amine organique avec un excès stoechiométrique de phosgène du produit brut gazeux obtenu dans la transformation, comprenant un premier dispositif de séparation (2), qui présente
- une conduite d'introduction de produit brut (3) pour un flux de produit brut gazeux contenant au moins l'isocyanate, du chlorure d'hydrogène et du phosgène qui n'a pas réagi,
- une première conduite d'introduction de liquide (4) pour un flux de liquide contenant au moins un liquide de refroidissement brusque et
- une première conduite d'évacuation de liquide (5) pour un flux de liquide contenant au moins une partie du liquide de refroidissement brusque et une partie de l'isocyanate,
une première conduite de gaz (9) partant du premier dispositif de séparation (2), pour le transport d'un flux de gaz contenant au moins du chlorure d'hydrogène, du liquide de refroidissement brusque vaporisé et du phosgène,
**caractérisé en ce qu'**au moins un dispositif d'ajout (11), pour l'introduction directe d'au moins un fluide de refroidissement pour une condensation et/ou une absorption au moins partielle du flux de gaz qui peut être conduit à travers la première conduite de gaz (9), étant associé à la première conduite de gaz (9).

2. Dispositif de nettoyage (1) selon la revendication 1, **caractérisé en ce que** le premier dispositif de séparation (2) et/ou la première conduite de gaz (9) sont disposés de telle façon que le flux de gaz qui peut être conduit à travers la première conduite de gaz (9) est dévié au moins une fois, préférablement au moins deux fois et particulièrement préférablement deux à cinq fois avant d'atteindre le dispositif d'ajout (11).

3. Dispositif de nettoyage (1) selon la revendication 1 ou 2, **caractérisé en ce que** le dispositif d'ajout (11) est constitué d'une ou plusieurs buses pour l'introduction directe du fluide de refroidissement dans le sens du courant ou à contre-courant, préférablement dans le sens du courant, ou est constitué de plusieurs buses qui sont positionnées dans la gaine de la première conduite de gaz (9), préférablement à des distances similaires le long de la circonférence de la première conduite de gaz (9) .

4. Dispositif de nettoyage (1) selon la revendication 1 ou 2, **caractérisé en ce que** le dispositif d'ajout (11) présente au moins une, préférablement plusieurs ouvertures pour la génération d'un rideau de fluide sur la section transversale entière de la première conduite de gaz (9), qui sont positionnées dans une partie de conduite s'étendant horizontalement dans la partie supérieure de la gaine de la première conduite de gaz (9), de sorte que le flux de gaz croise les lamelles ou les faisceaux du rideau de fluide.

5. Dispositif de nettoyage (1) selon l'une quelconque des revendications 1 à 4, **caractérisé en ce que** le dispositif d'ajout (11) présente une conduite d'introduction de fluide de refroidissement (12) pour le transport du fluide de refroidissement.

6. Dispositif de nettoyage (1) selon l'une quelconque des revendications 1 à 5, **caractérisé en ce que** la première conduite de gaz (9) conduit à un deuxième dispositif de séparation (10).

7. Dispositif de nettoyage (1) selon la revendication 6, **caractérisé en ce qu'**une deuxième conduite de liquide (13), pour le recyclage au moins partiel d'un flux de liquide en tant que liquide de refroidissement brusque, part du deuxième dispositif de séparation (10) et conduit au premier dispositif de séparation (2).

8. Dispositif de nettoyage (1) selon la revendication 7, **caractérisé en ce qu'**en outre des dispositifs de régulation (15a) et (15b), sont associés à la deuxième conduite de liquide (13) et à la conduite d'introduction de fluide de refroidissement (12), pour le contrôle des flux de quantité de liquide qui peuvent être conduits d'une part à travers la deuxième conduite de liquide (13) vers le premier dispositif de séparation (2) et d'autre part à travers la conduite d'introduction de fluide de refroidissement (12) vers le dispositif d'ajout (11) .

9. Procédé pour séparer un isocyanate organique préparé en phase gazeuse par transformation d'une amine organique avec un excès stoechiométrique de phosgène du produit brut gazeux obtenu dans la transformation, comprenant les étapes de,
(i) condensation au moins partielle du flux de produit brut, qui contient au moins l'isocyanate, du chlorure d'hydrogène et du phosgène qui n'a pas réagi, par mise en contact avec au moins un flux de liquide, qui contient au moins un liquide de refroidissement brusque, dans un premier dispositif de séparation (2) avec obtention d'un flux de liquide contenant au moins une partie du liquide de refroidissement brusque et une partie de l'isocyanate et
d'un flux de gaz contenant au moins du chlorure d'hydrogène, du liquide de refroidissement brusque vaporisé et du phosgène,
(ii) déviation du flux de liquide obtenu dans l'étape (i) par l'intermédiaire d'une première déviation de liquide (5) et du flux de gaz obtenu dans l'étape (i) par l'intermédiaire d'une première conduite de gaz (9) et
(iii) condensation et/ou absorption au moins partielle du flux de gaz dévié dans l'étape (ii) à travers la première conduite de gaz (9),
**caractérisé en ce que** la condensation et/ou l'absorption au moins partielle dans l'étape (iii) est réalisée par l'introduction directe d'au moins un fluide de refroidissement, le fluide de refroidissement étant introduit directement dans la première conduite de gaz (9) par l'intermédiaire d'au moins un dispositif d'ajout (11), qui est associé à la première conduite de gaz (9).

10. Procédé selon la revendication 9, **caractérisé en ce que** le rapport en poids du liquide de refroidissement introduit sur le flux de gaz conduit à travers la première conduite de gaz (9) est de 1 : 100 à 10 : 1, préférablement 1 : 12 à 1 : 1 et particulièrement préférablement 1 : 10 à 1 : 2.

11. Procédé selon la revendication 9 ou 10, **caractérisé en ce que** le fluide de refroidissement est choisi en fonction du solvant utilisé dans l'étape (i) en tant que liquide de refroidissement brusque.

12. Procédé selon l'une quelconque des revendications 9 à 11, **caractérisé en ce qu'**il comprend les étapes supplémentaires de
(iv) séparation du flux de gaz provenant de la première conduite de gaz (9) dans un deuxième dispositif de séparation (10), en un flux de liquide et un flux de gaz et
(v) recyclage du flux de liquide obtenu dans l'étape (iv) en tant que liquide de refroidissement brusque par l'intermédiaire d'une deuxième conduite de liquide (13) dans le premier dispositif de séparation (2), un flux partiel pouvant être détourné, préférablement étant détourné, comme fluide de refroidissement dans la conduite d'introduction de fluide de refroidissement (12) .

13. Procédé selon l'une quelconque des revendications 9 à 12, **caractérisé en ce que** le fluide de refroidissement est le chlorobenzène, l'o-dichlorobenzène, le p-dichlorobenzène, les trichlorobenzènes, les chlorotoluènes ou chloroxylènes correspondants, le chloroéthylbenzène, le monochlorodiphényle, le chlorure d'a-naphtyle ou de β-naphtyle, l'ester éthylique de l'acide benzoïque, les esters dialkyliques de l'acide phtalique, le phtalate de diisodiéthyle, le toluène et/ou le xylène, préférablement le chlorobenzène, l'o-dichlorobenzène et/ou le p-dichlorobenzène et particulièrement préférablement le chlorobenzène ou l'o-dichlorobenzène.

14. Procédé selon l'une quelconque des revendications 9 à 13, **caractérisé en ce qu'**une baisse de température dans le flux de gaz de 0,5 à 50 K, préférablement de 1 à 40 K et particulièrement préférablement de 3 à 30 K se produit par le dispositif d'ajout (11).
